# EUROPEAN PATENT SPECIFICATION

(11) **EP 4 182 013 B1**
(45) Date of publication and mention of the grant of the patent: **04.03.2026**
(21) Application number: 21740440.9
(22) Date of filing: 29.06.2021
(51) Int. Cl.: A61N 1/362, A61N 1/37

(54) **APPARATUS FOR MONITORING TEMPORARY PACING DEVICES**
VORRICHTUNG ZUR ÜBERWACHUNG VON TEMPORÄREN SCHRITTMACHERGERÄTEN
APPAREIL DE SURVEILLANCE DES DISPOSITIFS DE STIMULATION TEMPORAIRE

(30) Priority: 14.07.2020 GB 202010794
(43) Date of publication of application: 24.05.2023
(73) Proprietor: Ventripace Medical Ltd, Milton Park, Oxfordshire OX14 4SB (GB)
(72) Inventor: MEESE, Daniel, Wycombe Buckinghamshire HP11 2PJ (GB)
(74) Representative: Potter Clarkson
(86) International application number: PCT/EP2021/067884
(87) International publication number: WO 2022/012922

(56) References cited:
- US-A1- 2001 031 925
- US-A1- 2003 120 318
- US-A1- 2008 039 904
- US-A1- 2010 094 370
- US-A1- 2016 287 166
- US-A1- 2018 152 972

## Description

### FIELD OF THE INVENTION

The present invention relates to apparatus and methods for monitoring the operation of a temporary cardiac pacemaker, particularly but not exclusively in a human patient.

### BACKGROUND

Following open heart surgery, patients may experience heart rhythm disturbances which can lead to serious complications during recovery. To counter this, cardiac surgical patients are typically fitted with a temporary pacing system, or pacemaker, to regulate the heart's rhythm and ensure a near-normal cardiac output is maintained during recovery. Such systems work by "pacing", i.e. by delivering electrical impulses (or "pacing spikes") at predetermined energy levels to the specific locations on the heart at controlled intervals.

Post-surgical temporary pacing therapy is achieved (in most cases) by attaching a specialised pacing wire to either the epicardial (outer) surface of the heart or the endocardial (inner) surface of the heart, feeding the wire through the patient's abdomen (or out through a vein in the patient's leg or neck), and attaching the wire to an extension cable which is inserted into the temporary pacing device. Such pacing wires are known to be both unstable and subject to degeneration.

This instability, coupled with devices that lack the sophistication seen in permanent pacing, as well as device management being undertaken by the Intensive Treatment Unit (ITU) doctors and nurses who lack the expertise and experience of cardiologists and cardiac physiologists, can lead to a failure to identify acute and sudden changes in device function or patient underlying rhythm. As a result, cardiac output may be affected, resulting in higher use of inotrope (muscle contraction) medication and an extended and costly ITU stay. Other, more serious, consequences of a failure to recognise changes in pacing function can result in cardiac arrest and patient death.

Hospital audits regularly identify serious problems in the management of temporary pacing in the post-surgical ITU setting. These investigations report high incidences of failure to recognise loss of capture (the delivery of a pacing impulse that has insufficient energy to cause the heart to contract), oversensing (the pacemaker sensing a signal that is not a cardiac signal leading to inappropriate inhibition of pacing), undersensing (the pacemaker not sensing a cardiac signal and so continuing to pace when it should inhibit), poor pacing mode selection (the pacing mode instructs the pacemaker how it should function in relation to the patient's underlying cardiac rhythm) and other errors in programming, all of which can be extremely hazardous to patients.

Although patient safety is the paramount concern, questions also exist into the extent of how appropriate device optimisation (that is, ensuring that the system has the correct settings for the patient's underlying rhythm) can aid recovery. Studies have found that patients with intact and normal cardiac conduction across their atrio-ventricular node (the electrical junction between atria and ventricles) had better blood pressure when the device was optimised in such a way that it allowed the ventricles to contract without the use of pacing. It has also been shown that patients with correct mode selection experience a better clinical outcome.

Pacemakers generally come in two forms. Single chamber devices where only a single lead is used to place an electrode in either the atrium or the ventricle, or dual chamber where two leads (one in the atrium and one in the ventricle) work together. A third less common type of temporary pacemaker exists that uses a third lead which is attached to the left ventricle to enable the re-synchronisation of left and right ventricles in the context of heart failure.

Generally, pacemakers work using a timer based upon the base rate (the minimum rate the device will pace at) which is recorded in pulses per minute (ppm). When a conventional pacemaker sees a signal, that signal resets the timer of the pacemaker so the device does not deliver a pacing spike. In a single chamber device (single chamber modes are called AAI or WI depending on which chamber is being paced (i.e. atrial or ventricular)) set to pace at 60ppm, the timing window, which works in milliseconds (ms), will be 1000ms. Therefore, every 1000ms, provided that there has been no intrinsic beat to reset the timer (pacemakers are designed to hold back pacing in the presence of an intrinsic beat so as not to compete with the patient's own rhythm), the pacemaker will deliver a stimulus to make the heart contract. In a dual chamber system set to the same base rate, two timers run in sequence, the Atrial-Ventricular timer (A-V timer) and the Ventricular-Atrial timer (V-A timer) to generate contractions between the top and bottom chambers, resembling a normal heartbeat. The A-V timer is usually programmed at 200ms, but this number is adjustable. The V-A timer is set at the remaining time after the A-V timer has been subtracted from the base rate. So, in this case, 800ms. Again, should an intrinsic signal be seen in either chamber, then the timer for that chamber will reset and the device withholds pacing for that chamber.

Bi-ventricular pacing is another feature of pacing that is important, albeit less common. The need for bi-ventricular pacing exists in patients suffering from heart failure. Heart failure is caused by both right and left ventricles beating out of time with each other. Bi-ventricular pacing is used to resynchronise ventricles back towards near normal function and thus reduce the effects of heart failure; this form of pacing is achieved using a further timer within the V-A timer known as the V-V timer.

Because a temporary pacemaker is usually fitted to a patient for a limited time only, monitoring of the operation of the temporary pacemaker and of the patient's physiology are usually carried out by an experienced person, such as a nurse or doctor; the attention such people can give any one patient is limited, but this does not significantly detract from the attention they are able to give to all of their patients because the situation is temporary. In time it is normally hoped that the patient's condition and cardiac function will settle down to a routine operation, or if not a more permanent pacemaker can be considered. Nevertheless, monitoring of a temporary pacemaker does require attention by personnel specifically trained to deal with cardiac matters (because of the gravity of cardiac problems) but also with the complex interaction between pacing devices and the heart. The need for monitoring personnel to be trained in this latter aspect means that many otherwise experienced medical personnel (i.e. doctors and nurses without specific training/experience) are not well-equipped to monitor patients with temporary pacemakers. Moreover, there are a variety of different problems which arise with temporary pacemakers that can be hazardous to the patient, these include:
- Oversensing - The pacing device sees a signal that is not cardiac in nature, but responds to it by withholding pacing
- Undersensing - The pacing device fails to see an intrinsic heart signal (from either atria or ventricles) and thus continues to pace when not required
- Loss of Capture - The pacing device has delivered an impulse at the correct time, but the impulse is of an insufficient energy to cause the heart to contract
- Mode selection - incorrect mode selection based on the patients underlying rhythm can lead to suboptimal haemodynamics, an increased use of inotropes and a prolonged ITU stay

The above problems are particularly acute for patients whose pacemaker is only temporary, and may also occur (albeit less frequently) in patients with long term pacemakers. In addition, patients with temporary pacemakers can display problems which are common with long term pacemakers, such as arrhythmia.

United States Patent Application US 2008/039904 A1 describes an intravascular implantable system for providing electrical stimulation of tissue inside an animal to deal with a clinical condition. The system comprises a power supply module supplying energy to the implantable system, an implanted control module controlling operation of the implantable system and producing desired digital waveforms.

United States Patent Application US 2018152972 A1 describes a method initiating a communication link between an external instrument (EI) and an implantable medical device (IMD), established a first connection interval for conveying data packets between the El and IMD and monitors a connection criteria that includes at least one of a data throughput requirement.

### SUMMARY OF THE INVENTION

The invention is defined in apparatus claim 1 and computer readable medium claim 13. Further aspects and preferred embodiments are defined in the appended claims.

Disclosed herein is a means for monitoring temporary pacing devices which directly monitors the signals passing to and from the heart and from the temporary pacemaker can not only relieve the need for the monitoring of such devices to be overseen by specifically trained personnel, and address the problems associated with both temporary and long term pacemakers, but also simultaneously provide monitoring and diagnostic functionalities which are usually the preserve of the more experienced doctors, specialists or surgeons. This reduces the amount of attention a cardiac patient requires from such a senior medic and allows purely short term and non-critical disturbances in cardiac and/or pacing device operation to be normalised automatically but more serious disturbances to be alerted for the appropriate human intervention.

The present invention provides a temporary pacing management and safety, TPMS, monitoring apparatus adapted to monitor a cardiac pacing device that is located externally to a patient's body, particularly a temporary cardiac pacing device, which is connectable to the patient's heart via epicardial or endocardial leads, the TPMS monitoring apparatus comprising:
a signal acquisition module adapted to acquire via the electrical connections with the heart and pacing device, cardiac signals indicative of cardiac operation, pacing impulses emitted by the pacing device, evoked signals emitted from the heart in response to the pacing impulses, and any unidentified noise signals, the electrical connection with the heart comprising the epicardial or endocardial leads;
a processor adapted to receive from the signal acquisition module and to analyse the cardiac and evoked signals, the pacing impulses and any noise signals;
a data store,
wherein the processor is adapted to:
   i. establish a base level operation of the heart and pacing device and to store the associated quality, size and/or timing values of the cardiac and evoked signals and the pacing impulses in the data store;
   ii. receive instantaneous values of quality, size and/or timing values of the cardiac and evoked signals and the pacing impulses;
   iii. compare the instantaneous values against the values in the data store to establish differences therebetween;
   iv. analyse:
      a. any noise signal received,
      b. the instantaneous values received at step ii above, and
      c. any difference(s) at step iii above in terms of its/their quality size and timing, and
   v. raise an alarm in the event a noise signal occurs and/or no cardiac or evoked signal is received.

Such an arrangement is capable of being set up by an appropriately-trained medic, nurse or allied health professional, who can establish that the base operation of the heart and the pacing device is correct and neither is operating abnormally in any observable way. The apparatus can then be allowed to monitor both cardiac function and pacemaker operation without further operator input or attention being required, unless something untoward occurs and an alarm Is raised (the alarm can be both shown on the display (or User Interface) and by an audible or other visual alert, it could also be communicated to a secondary device at a remote location such as to a medic or nurse who Is not in the patient's vicinity, such as by WiFi or Bluetooth to that person's mobile phone or other internet-enabled device or User Interface (UI), for example). It is also possible for the apparatus at the analysis step iv to discriminate between the types of noise signals and to give an indication of oversensing when appropriate, to assess the significance of the instantaneous values received at step ii for mode selection purposes or for sensing arrhythmia and, when no evoked signal is sensed following a pacing impulse, to determine if this is due to loss of capture, when pacing timer is not reset when an intrinsic heart signal is present to give an indication of undersensing and to assess the significance of the qualitative, size and/or timing difference(s) for mode selection purposes or for sensing arrhythmia.

The processor may be adapted to operate according to predetermined algorithms, such as those described below, to monitor a temporary pacing device for Its basic operation, to monitor for oversensing, undersensing, loss of capture, far field oversensing, and arrythmia. An advantage of this is that all of the algorithms can run concurrently or simultaneously, and in any combination either simultaneously or concurrently (in certain circumstances it may be desirable to monitor one, two or more functions whilst other functions may be less important (or less likely to occur in a particular patient) and so these can be monitored at a reduced frequency, thus allowing the associated algorithms to be run in the appropriate manner and frequency).

The processor may also be adapted to respond to operator inputs to carry out QT analysis of cardiac function, arid cardiac rhythm analysis in order to suggest adjustments to the pacing device operating parameters, according to other predetermined algorithms such as those described below. The processor may be adapted to record data for real-time or offline analysis. The processor may also provide optimisation suggestions based on this analysis.

The data store may be adapted to contain causal data and values relating to the causes of cardiac arrythmia and related pacing device settings appropriate for each cause, and the processor may be adapted when requested to perform rhythm analysis to compare instantaneous values with those in the data store, identify the cause which best matches the causal data and values in the data store and to display related pacing device settings as a suggested optimisation change, so that the patient receives the most appropriate pacing impulses from the pacing device. The data store may contain pre-recorded electrograms and predetermined timings and values associated with different cardiac functions and/or malfunctions, and the pacing values associated therewith.

The apparatus may incorporate a communications module, which can not only communicate alerts to secondary devices but also allow remote configuration of the apparatus, and the raising of alerts and monitoring remotely. The user interface (UI) for this can be via a communications channel to a remote UI. Additionally or alternatively, the apparatus may provide the following functionality:
Comparison of the timings of pacing impulses to the settings of the monitored device and assessment of how appropriate that timing is.
Ability to sense the evoked response generated by the pacing impulse to the epicardium/myocardium to establish that the impulse captured the heart.
Comparison of the timings of pacing impulses to those of intrinsic intracardiac electrograms (EGM) and to recognise if the monitored device has sensed or under-sensed the intrinsic heartbeat.
Use intracardiac electrograms from both atrial and ventricular pacing leads to assess and diagnose patient's underlying rhythm.
Use intracardiac electrograms to recognise changes in patient's underlying rhythm.
Establish the difference between intrinsic heart rhythm and noise.
Recognise onset of rhythms, determine if the monitored device was at fault and save the electrograms for further analysis.
Use intracardiac electrogram timings from both atrial and ventricular signals to recommend optimal pacing parameters for the patient.

The apparatus may also be able to integrate data from additional biomedical sensors, and/or may be able to send live or recorded data to a secondary device in a processed or unprocessed state.

### BRIEF DESCRIPTION OF THE DRAWINGS

The invention will now be described by way of example and with reference to the accompanying figures, in which;
Figure 1 is a schematic illustration of a temporary pacing management and safety (TPMS) monitoring device connected to a monitored device and a heart via epicardial leads;
Figure 2 is a block diagram of an in-line connected TPMS monitoring device;
Figure 3 is a block diagram of a tap-off connected TPMS monitoring device;
Figure 4 is a functional block diagram of a TPMS monitoring device;
Figure 5 is a flow chart for a pacing function monitor (VVI/AAI mode);
Figure 6 is a flow chart for pacing sensing analysis to detect noise leading to oversensing for single chamber modes (VVI/AAI mode) and dual chamber mode (DDD);
Figure 7 is a flow chart for pacing undersensing;
Figure 8 is a flow chart for confirmation of capture;
Figure 9 is a flow chart for pacemaker sensing analysis to far-field signals leading to oversensing for dual chamber mode (DDD);
Figure 10 is a flow chart for QT interval analysis;
Figure 11 is a flow chart for rhythm analysis and optimisation;
Figure 12 is a flow chart for detecting cardiac arrhythmia, and
Figure 13 is an illustration of a TPMS monitoring device.

### DETAILED DESCRIPTION OF THE EMBODIMENTS

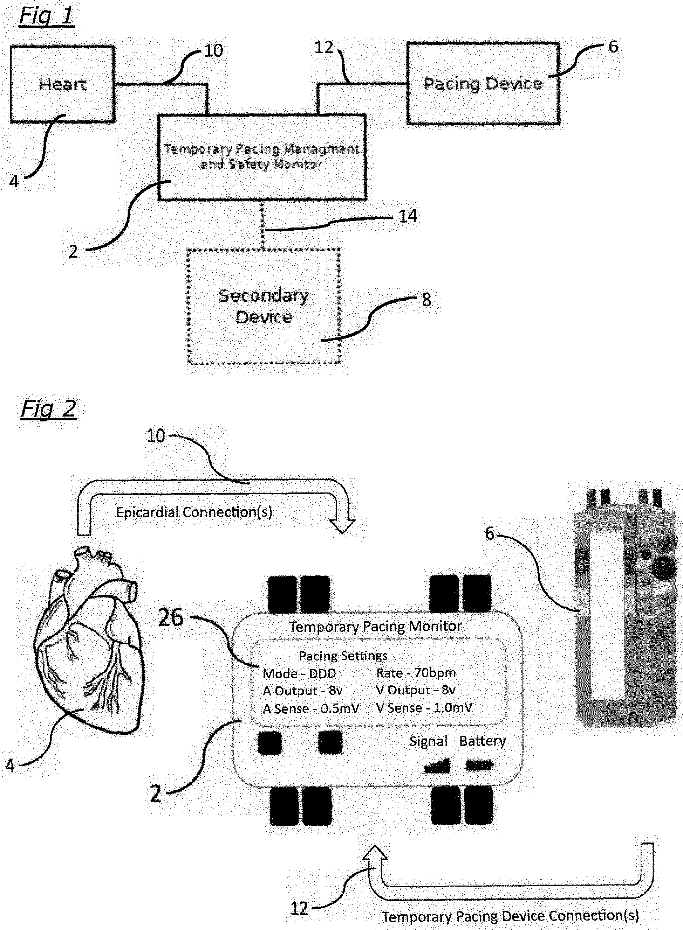

Figure 1 shows a temporary pacing management and safety (TPMS) monitoring device 2 connected to a heart 4, a pacing device 6, or pacemaker, which is to be monitored, and a secondary device 8. The TPMS device 2 is connected electrically to the heart 4 directly via epicardial or endocardial leads 10 (wherever reference is made herein to epicardial leads it should be understood that these could alternatively be endocardial leads, or a mixture of both). The monitored pacing device 6 is external to the body of the patient and is connected to the TPMS monitoring device 2 via additional electrical leads 12. The secondary device 8, which is a smartphone or some other remote electronic device, is connected to the TPMS monitoring device 2 via a communication link 14, which may be a wireless link or a hard-wired link.

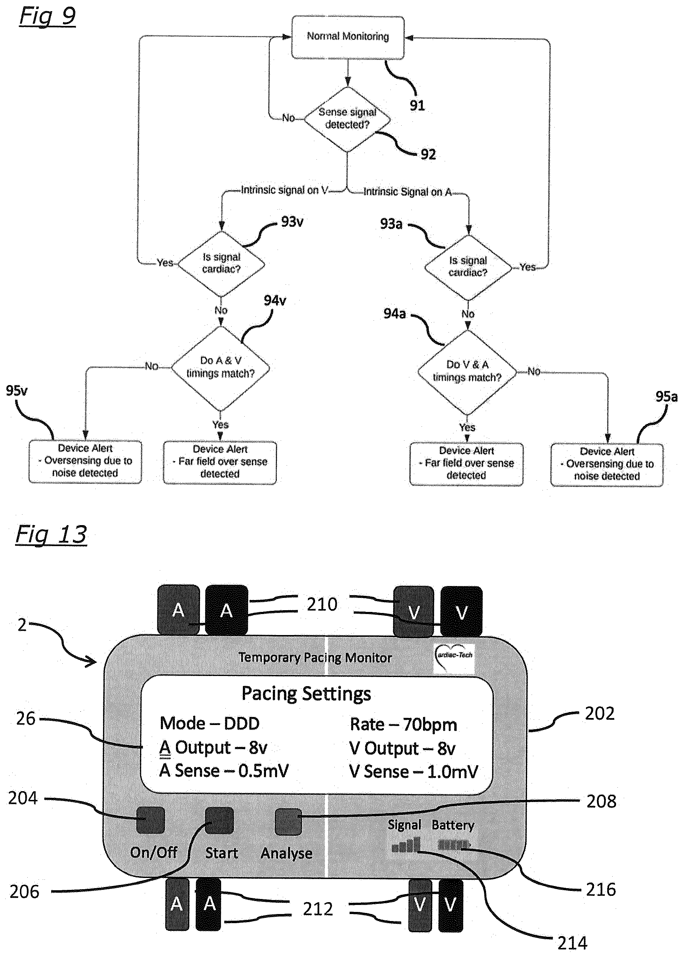

Figure 2 shows an inline arrangement of a TPMS monitoring device, in which one or more epicardial connections 10 lead to the patient's heart 4 from the TPMS monitoring device 2, and a temporary pacing system 6 is connected to the TPMS monitoring device 2, so that the three elements are connected inline. This inline arrangement is electrically similar to the tap style (described below), but offers the additional benefit of impedance measurement, which gives a greater diagnostic capability. The TPMS monitoring device 2 has a display, or local user interface, 26 (shown more clearly in Figure 13) indicating the pacing mode (as DDD, or dual chamber mode), the pacing rate (shown as 70bpm - the same as 70ppm) the atrial and ventricular output voltage (both shown as 8V) and the atrial and ventricular "sense" (i.e. the sensitivity threshold) shown as 0.5mV and 1.0mV respectively).

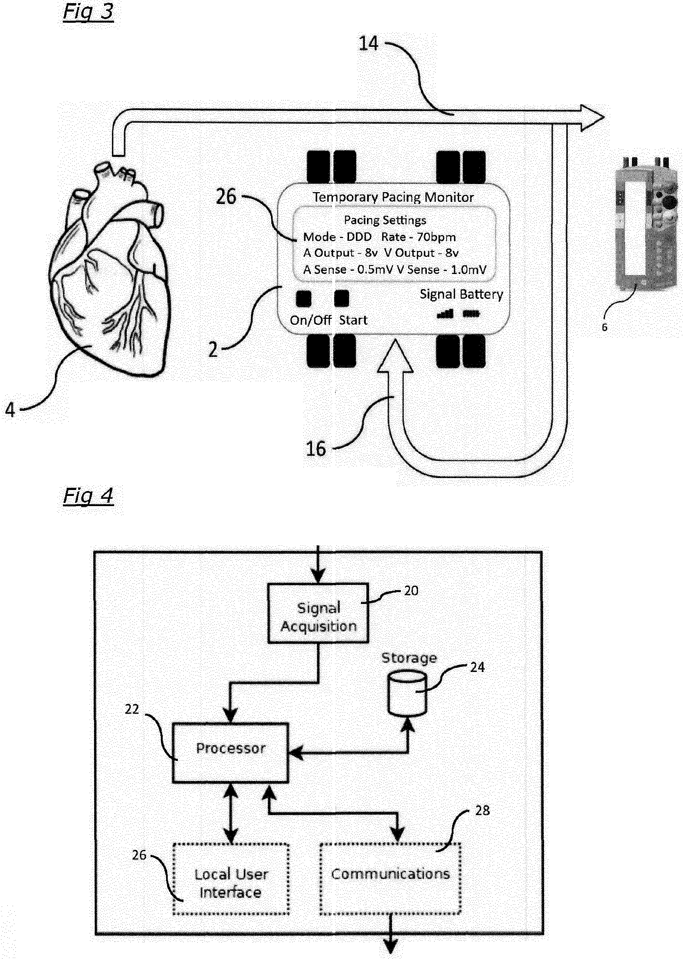

Figure 3 shows an alternative, 'tap-off' style wiring arrangement for the TPMS monitoring device, in which there is a direct connection 14 (comprising one or more epicardial connections) linking the temporary pacing system 6 to the heart 4, and the TPMS Monitoring device 2 is connected by a lead 16 to the connection 14. This tap-off arrangement functions electrically in a similar fashion as does the inline arrangement, and it allows simpler wiring, but measurement of impedance or signal loading with the tap-off arrangement is not as straightforward.

Figure 4 shows a functional diagram of a TPMS Monitoring device 2, comprising a signal acquisition module 20, a processor 22, a data storage module 24, a local user interface 26, and a communications module28. The local user interface and the communications module are shown in shadow because either or both may be incorporated in different arrangements.

The signal acquisition module 20 provides the physical connection for the electrical signals entering the processor 22. It is designed to capture two types of signals, the epicardial signals from the heart and the pacing signals from the monitored device. The signal acquisition module 20 and processor 22 monitor all signals for noise or unexpected shape, and compare measured impedance with expected values from a database (held either in the data storage module 24 or elsewhere in the TPMS monitoring device 2) in order to ensure functional safety and to maintain signal integrity. Degenerated signals, missing signals, and/or incorrect signals are therefore detected and an appropriate response/alert is provided.

The processor 22 is responsive to signals provided by the signal acquisition module 20 and is adapted to compare the output parameters of the monitored pacing device 6 and the parameters of the signals from the epicardial leads. The signals are subjected to parametric and algorithmic analysis. A parameter change generates an audio-visual response or alarm on the TPMS device 2, and/or on a secondary device. The signals are stored in the data storage module 24. The signals are displayed on the local user interface 26, when present. The signals are transferred outside the TPMS monitoring device 2 via the communications block 28, when present. It will be appreciated that signals from the epicardial leads 10, 14 (see Figures 2 and 3) and also signals from the monitored device 6 are available for processing. The local user interface 26 allows the display of real-time events, alarms, and other information to the user, such as the parameters shown in Figures 2 and 3. It may incorporate controls (not shown) to allow parameters of the TPMS monitoring device 2 to be adjusted. The communications module 28 allows transfer of data from the TPMS monitoring device 2 to a secondary device, such as a smartphone or other remote device.

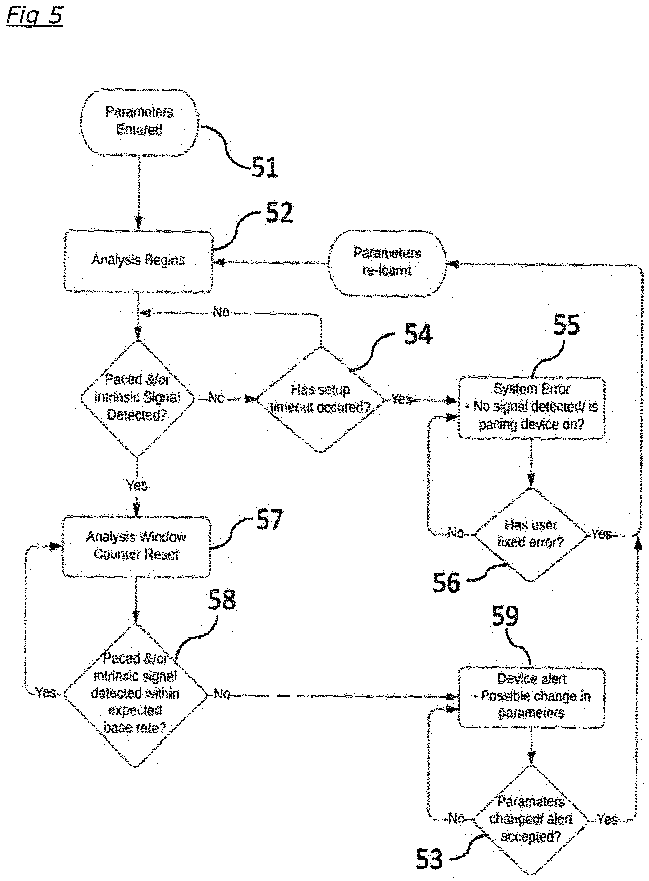

Figures 5 to 11 describe algorithms that the TPMS monitoring device 2 may incorporate. These algorithms may be realised procedurally, as DSP (digital signal processing) techniques or by other signal processing methods known in the art, including Fast Fourier Transforms (FFT), correlation techniques and convolution transforms.

Figure 5 shows the operation of the TPMS monitoring device 2 during normal (non-error) conditions. After parameter setup 51 (parametric input can be manual or automatic) the device starts a control loop step 52 and waits for a signal from the pacemaker or from the patient. If no such signal arises, a timer step 54 either repeats this loop or if too much time has passed, causes an alert step 55 that can be cleared by the operator at step 56. The operator may then be invited to alter parameters at step 57 if he sees fit. If a valid signal is received, a continuous loop is set up comprising timer step 57 and comparator step 58; this loop monitors the operation of the system to ensure it is within healthy or desired parameters continuously. Should signals be received outside of acceptable time windows, then another alert step 59 is activated and the user may again be invited to change parameters at step 53, at which point the loop reverts to the main control loop at step 52.

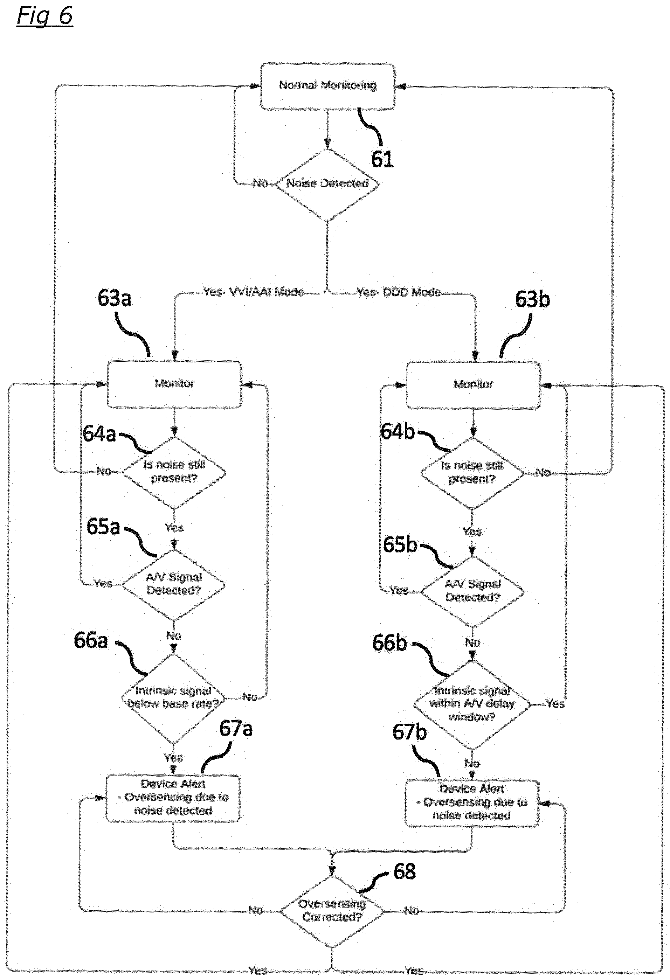

Figure 6 shows a flowchart for detecting oversensing of noise, in both single chamber mode (i.e. VVI/AAI mode) 63a and dual chamber mode (DDD) 63b, by the monitored device 6. Once monitoring has been initiated at step 61, if no noise is detected, normal monitoring continues as described in Figure 5 above.

If the pacemaker is operating in single chamber mode and noise is detected by the monitoring and safety device, the algorithm begins looking for paced impulses and/or intrinsic cardiac signals within the pacing timing window. This is performed at steps 64a, 65a and 66a. If no such paced impulses are seen, or intrinsic cardiac signals are detected which are slower than the device base rate (slower than the timing window), the device will issue an alarm at step 67a. If it is determined at step 64a that the noise has disappeared, the algorithm reverts to normal monitoring by entering the control loop at block 61a. If noise is detected on the atrial lead in DDD mode at step 64b, the same response as the single chamber mode applies. If noise is detected on the ventricular lead by the monitoring and safety window whilst the pacemaker is in DDD mode, the algorithm looks for an intrinsic cardiac signal at the atrio-ventricular delay (AV delay) step 66b. Should no response be seen, the device will alarm at step 67b. This function is performed in a similar way to the process for single chamber mode. In either mode a decision step 68 determines if the oversensing issue has been corrected (such as by an adjustment of parameters or rearrangement of wiring) and then reverts to the previous loop at 63a for single chamber mode or at 63b for dual chamber mode.

When a conventional pacemaker sees a signal, that signal resets the timer of the pacemaker and thus the device does not deliver a pacing impulse. However, the pacemaker has no knowledge of what that signal is other than the fact that it exists. Apparatus in accordance with the invention can establish if that signal was a real cardiac signal (i.e. the device has behaved appropriately) or an extracardiac signal such as noise and thus has behaved inappropriately. At this point, the apparatus will alert the inappropriate behaviour to the nurse in charge of the patient and/or the medic responsible for the patient.

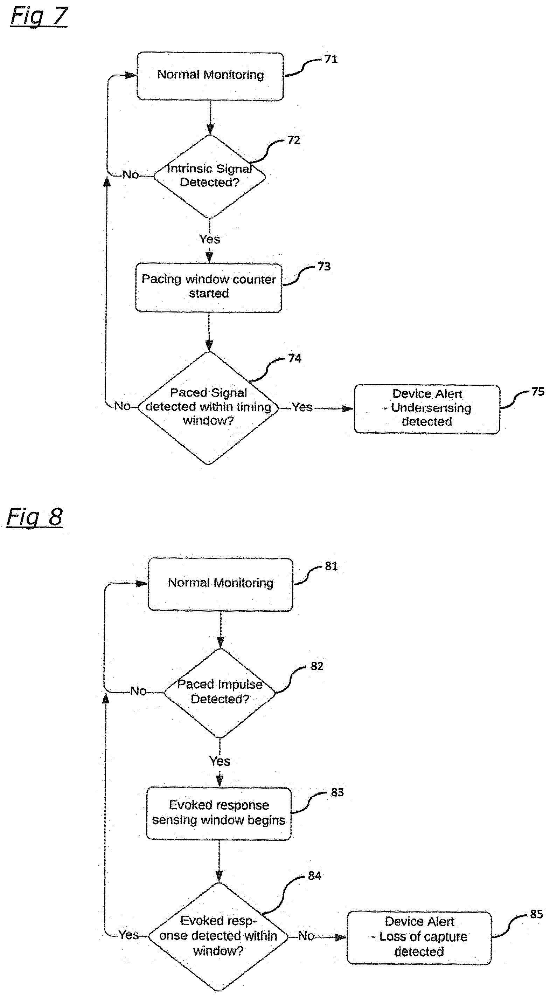

Figure 7 shows a flowchart for detecting undersensing in both atrial and/or ventricular channels. The algorithm starts at step 71 and waits until an intrinsic (patient generated) signal occurs, at step 72. If such a signal does occur, a timer step 73 is started and step 74 looks to see if an undesirable pacing signal occurs within a certain time or not (looking if there is a signal which the pacemaker did not see, resulting in an unnecessary paced impulse). If not then the loop continues back to step 71. However if an undesirable pulse is picked up, an appropriate alert is issued at step 75. The foregoing describes an algorithm for detecting undersensing in VVI, AAI, and DDD mode. When an intrinsic heart signal is seen by the main unit from either the atrium or the ventricles, a timing window is triggered that is set to the timing set up of the pacemaker. The device then watches to see if a pacing impulse is delivered inside of this window. Should a pacing impulse be detected to be delivered, this would be considered inappropriate operation of the system and the device would alert.

Figure 8 shows an algorithm for confirming capture by the monitored pacing device 6, that is, that the pacing device has delivered an impulse at the correct time and energy level for the heart to contract as required. The algorithm is started with an onset step 81, a decision step 82 waits for a paced impulse to be detected. Once a paced impulse has been detected the algorithm moves on to the evoked response window step 83. A decision step 84 waits for an evoked response signal to be detected within the window step 83, indicating whether or not the patient's heart has responded to the paced impulse. If an evoked response is detected within the window, the algorithm flow returns to waiting for a paced impulse at step 82. If no evoked response is detected within the window, the device alerts at step 85. The foregoing describes an algorithm for confirming capture on a monitored device 6. When a pacemaker delivers an impulse, the amount of energy used needs to be enough to trigger a heartbeat. Due to factors such as position of the epicardial lead relative to the heart, the health of the tissue the lead is attached to, and/or the quality of the contact of the lead with the heart muscle, the amount of energy required can differ for different patients. Furthermore, in temporary pacing lead parameters can change: either suddenly due to the lead moving, or slowly over time as the heart begins to endotheliorise (grow tissue) over the lead. These changes can lead to loss of capture - the amount of energy delivered is not enough to stimulate a heartbeat.

The TPMS monitoring device 2 will be able to establish capture of a paced impulse by looking for something called the evoked response. This is a signal generated by the heart muscle as the impulse causes a contraction. Once the device senses a paced impulse from the pacemaker, a window starts that looks for the evoked response. If it is seen, the device does nothing. If it fails to see the evoked response, the device alerts.

Figure 9 shows an algorithm for detecting farfield oversensing, in single chamber WI and AAI modes, by the monitored pacing device 6. The algorithm starts with an onset step 91 (for the continuous monitoring of pacing function, as described in connection with Figure 5), and a decision step 92 will loop until a sensed signal is detected. When a signal is detected at step 92 a new branch is started according to whether the signal is atrial or ventricular, a further decision step 93v/93a confirms if the signal has the correct components for a near field signal (and thus from the correct chamber) or if it is deemed to be non-cardiac. If it is deemed to be near field the algorithm loops back to normal monitoring 91, if it is deemed to be non-cardiac then decision step 94v/94a assesses whether the non-cardiac signals have the same timing as the paced/sensed signals in the opposite chamber. If not, an alert is given at 95v/95a for oversensing of noise; if yes, the algorithm moves to give an alert for oversensing of farfield signals at 95v/95a. This algorithm is applied to both atrial and ventricular channels simultaneously. The foregoing describes an algorithm for detecting oversensing due to far-field signals (signals from one pacing lead detected on the opposite pacing lead) in dual chamber mode only (this kind of oversensing cannot be seen in single chamber mode). If an intrinsic signal is seen on either A or V channels, the device assesses the signal for cardiac components (near-field signals display cardiac components, far-field do not). Should it be confirmed that the signal does not have the relevant cardiac components, the safety monitor compares the A and V channel to see if the signals occur simultaneously. If they do, the device alarms for far-field over sensing. If they do not, the device alarms for oversensing of noise.

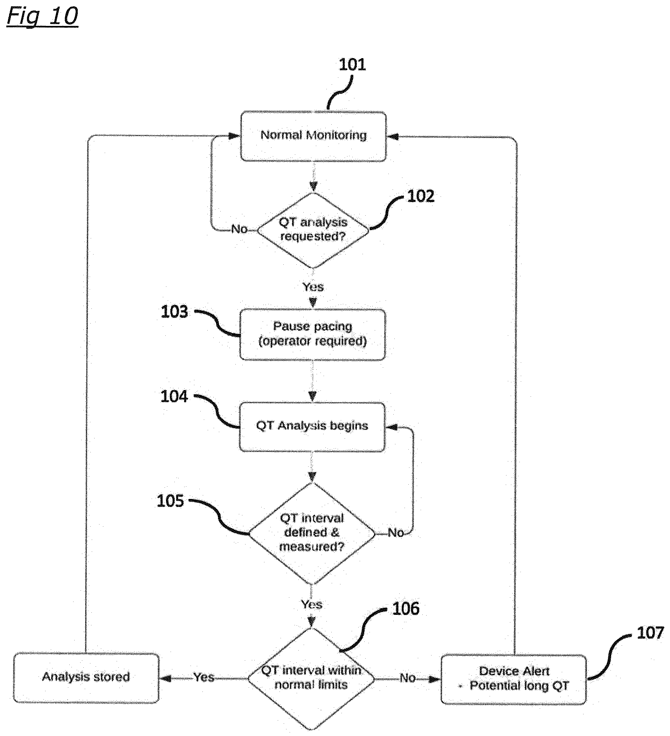

Figure 10 shows an algorithm for QT analysis (the QT interval is the interval between the onset of the ventricular contraction (depolarisation) phase and the end of ventricular resetting (repolarisation) phase). The analysis begins with QT analysis being requested at step 102 during normal monitoring 101, and begins (after an option at 103 to pause the system) at 104 with the sampling of a number of beats to determine the current QT interval. After measurement of the QT interval 105 a decision step 106 checks if the determined QT interval is within limits. If the QT interval is within limits the analysis continues on a beat to beat basis 104 over a defined number of beats, if the QT interval is not within limits the device alerts 107. The foregoing describes a software algorithm for QT analysis. The QT interval is defined as the total time an impulse takes to pass through the ventricles and then for the ventricular tissue to 'reset' itself ready for the next contraction. The total time for this to occur usually falls between 420ms and 460ms. Patients whose QT interval is longer than this are considered at risk of dangerous ventricular arrhythmia. The QT interval can be changed by certain medications frequently given in the post-surgical setting and so detailed analysis of the QT interval would be beneficial for medical teams when making decisions about courses of treatment.

The QT analysis algorithm works by measuring the patient's QT interval at the onset of monitoring. It does this by measuring beats (either intrinsic or paced) and looks for a trailing signal that can be defined as the T wave. It then measures the interval between the earliest indication of contraction (in a paced beat this would be the evoked response) to the last measurable point of the T wave. If the device finds it at this point to be outside of the normal intervals, it may alert. If however, the QT interval falls within normal measurements, the device continues to measure the signal on a beat to beat basis. Should prolongation of the QT interval be seen, the device may alert.

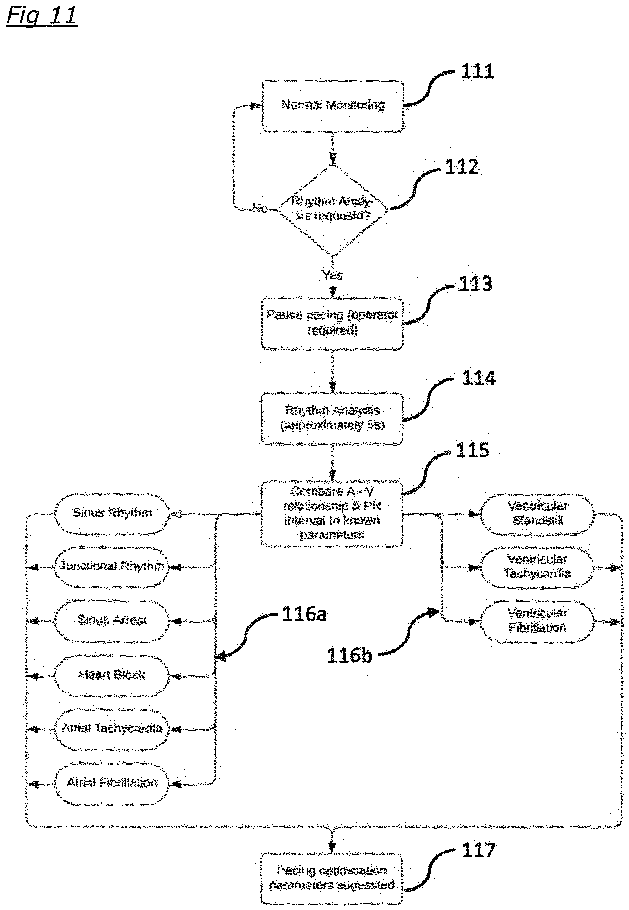

Figure 11 shows a schematic overview of an algorithm for rhythm analysis and optimisation. During normal operation 111 a request for rhythm analysis is made at 112 and the analysis begins (after an option at 113 to pause the system) at step 114. Analysis of the rhythm is performed and, once analysis is complete, the A-V relationship between the samples is established at step 115. A database of rhythm sequences 116a, 116b is used as a comparison source. The closest match to the analysed sequence is found and the results are fed back at step 115 to the device operator as targeted suggestions 117 for modifying the settings of the monitored pacing device 6.

For bi-ventricular devices, the optimisation process includes measuring the timings between right ventricular contraction and left ventricular contraction (V to V interval), so as to calculate the required settings for appropriate re-synchronisation.

The above overview of Figure 11 describes an algorithm for rhythm analysis to assist mode selection. Published data has shown that suboptimal pacing device programming may lead to reduced haemodynamics, increased use of inotrope medication and increased ITU stay. For example, a patient who is found to be in a rhythm called complete heart block (the impulses generated in the atrium are unable to transmit down to the ventricles leading to dissociation between the top chambers and the bottom chambers) but is only programmed to pace single chamber (ventricles only) will have a reduction in haemodynamics by as much as 30% of their cardiac output. This is due to the contractions of the atrium (which accounts for the 30% of blood moving through the heart) being out of sync with the contractions of the ventricles. By programming the pacemaker into dual chamber mode, the synchronicity of the top chambers to the bottom is restored and thus cardiac output is restored to 100% of the patient's normal volume.

For the analysis to be undertaken, a pause in pacing is required (this needs to be done on the pacemaker itself by reducing the pacing rate or pausing pacing function). To account for this and to reduce the risk of incorrect rhythm analysis, a 3 second delay in the algorithm is initiated to allow for the pause in pacing to take place. Once this has happened, a 5 second analysis window allows the device to analyse and record the relationship between signals coming from the top and bottom chambers. It then feeds this information into a rhythm database and compares and highlights sequencing that closest matches the recorded sequence before feeding back to the operator the diagnosed rhythm. Based upon its findings the temporary pacing management and safety monitoring device may also provide programming suggestions to best optimise the pacing device for the patient.

To allow for pacing optimisation, the device may use the same algorithm as seen for the rhythm detection algorithm (Figure 11) but with an added layer. A and V relationship is still assessed in the same way as described above, however as part of the optimisation the algorithm measures timings between the top and bottom chambers (known as the PR interval) to optimise AV delay at step 115.

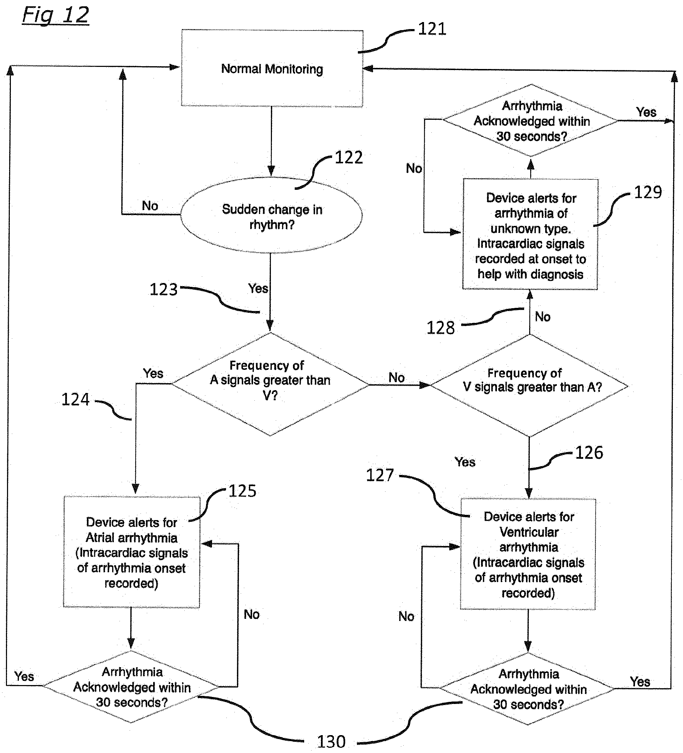

An algorithm for detecting cardiac arrhythmia is shown in Figure 12. At the start of the algorithm, during normal operation 121 of the pacing device, the TPMS monitoring device detects 122 a sudden change in cardiac rhythm. There is then a wait to detect the frequency of A (atrial) signals compared to the frequency of V (ventricular) signals, at which point 123 the algorithm establishes which of these signals is more frequent. Should a sudden change in rhythm occur and the frequency of the A signals be greater than that of the V signals 124 a data snapshot of the arrhythmia onset is taken and an alarm is raised 125 indicating atrial arrhythmia. The operator is then required at step 130 to confirm the presence of the arrhythmia within 30 seconds. Failure to do so and a second alert is triggered. The process is repeated until confirmation of the arrhythmia's presence. If a sudden change in rhythm occurs and the frequency of the V signals is greater than the A signals 126 a data snapshot of the arrhythmia onset is again taken and an alarm is raised 127 indicating ventricular arrhythmia. Again, the operator is required at step 130 to confirm the presence of the arrhythmia within 30 seconds. If this is confirmation is not given in time a second alert is triggered. As before, the process is repeated until confirmation of the arrhythmia's presence. In the case where there is no difference in frequency between the A and V signals 128, a data snapshot of the arrhythmia onset is again taken and an alarm is raised 129 indicating the presence of an unknown arrhythmia. Again the operator is required to confirm the presence of the arrhythmia within 30 seconds. If this is confirmation is not given in time a second alert is triggered. As before, the process is repeated until confirmation of the arrhythmia's presence. If a return to A-V synchrony is detected the device may offer the user a set of suggested optimised parameters for the monitored pacing device. The device may also use electrogram template and direction analysis to identify arrhythmia originating in the ventricle. This will be used where origin of the arrhythmia is unclear.

Figure 13 shows the TPMS monitoring device 2 in greater detail; the device receives signals from one or more I/O connections, these signals come from two sources: epicardial signals from the heart, and pacing signals coming from the monitored device. Algorithms implemented in the main unit firmware are needed to allow information to be extracted from these signals, extracted information is further used to alert the user or to provide feedback on possible adjustments to the monitored device that may be desirable. User feedback is provided to a local user via the display 3 (which may be a "touch screen", or there may be controls, switches or the like) or to a remote user via the communications module 28, and these means may also be used to adjust variables that control behaviour of the algorithms. The display 26 provides a digital display of the pacing settings. The device has a hardened outer casing 202 made of plastic. There is an On/Off power button 204, a Start monitoring button 206 and an Analyse button 208. There are inputs for pacing leads 210 and for epicardial leads 212; these may be insulated collets that can be tightened or loosened to accept and hold or release the leads as required. There are also displays to indicate the WAP/Bluetooth signal strength 214 and the battery level 216. The device includes an alarm (not shown) which can be in the form of an audible signal and/or a visual signal, by way of a loudspeaker, buzzer, flashing light or the like.

It will of course be understood that many variations may be made to the above-described embodiment without departing from the scope of the present invention. For example, in some embodiments of the invention further algorithms may be added to encompass addition pathologies and their associated signal parameters. It will be appreciated that there may be more epicardial leads and monitored device connections than shown in the drawings. Figure 12 is an illustration of a possible implementation of the invention. The implementation will vary depending on the embodied aspect of the invention.

It will be appreciated that it is straightforward to modify the apparatus and algorithms described above for bi-ventricular pacing (all of the pacing functionality described above can be provided by existing bi-ventricular pacing devices, which can also pace left ventricle and right ventricle independently of each other (known as V to V offset)), and that all of the monitoring features described above can be provided in bi-ventricular format simply by including a left ventricular arm, where appropriate, into each algorithm.

There may be controls provided on the TPMS monitoring device to allow differential setting of the various algorithms, to allow an operator to set which algorithms are to run simultaneously and/or at what frequency, and to set other algorithms to run concurrently and/or at a different frequency.

Where different variations or alternative arrangements are described above, it should be understood that embodiments of the invention may incorporate such variations and/or alternatives in any suitable combination.

## Claims

1. A temporary pacing management and safety, TPMS, monitoring apparatus (2) adapted to monitor a temporary cardiac pacing device (6) that is located externally to a patient's body, the TMPS monitoring apparatus being connectable to the patient's heart (4) via epicardial or endocardial leads (10, 14), the TPMS monitoring apparatus being located externally of the patient's body and comprising:
a signal acquisition module (20) adapted to acquire via electrical connections with the heart and the pacing device, cardiac signals indicative of cardiac operation, pacing impulses emitted by the pacing device, evoked signals emitted from the heart in response to the pacing impulses, and any unidentified noise signals, the electrical connection with the heart comprising the epicardial or endocardial leads;
a processor (22) adapted to receive from the signal acquisition module and to analyse the cardiac and evoked signals, the pacing impulses and any noise signals; and
a data store (24);
wherein the processor is adapted to:
i. establish a base level operation of the heart and pacing device and to store the associated quality, size and/or timing values of the cardiac and evoked signals and of the pacing impulses in the data store;
ii. receive instantaneous quality, size and/or timing values of the cardiac and evoked signals and of the pacing impulses;
iii. compare the instantaneous values against the values in the data store to establish differences therebetween;
iv. analyse any difference(s) at step iii above in terms of its/their quality, size and timing and:
in the event a noise signal is received, to follow a first predetermined algorithm to determine whether or not there has been oversensing, and/or
in the event that no cardiac or evoked signal is received following a pacing impulse, to follow a second predetermined algorithm to determine whether or not there has been loss of capture; and
v. raise an alarm if it determines that there has been any oversensing and/or any loss of capture.

2. A TPMS monitoring apparatus according to Claim 1 in which the processor (22) is adapted to follow a third predetermined algorithm when an inappropriately timed pacing signal is seen in relation to a cardiac signal to establish that there has been undersensing before an alarm is raised.

3. A TPMS monitoring apparatus according to any preceding claim in which the processor (22) is adapted to follow a fourth algorithm to sense arrhythmia, and to raise an alarm when arrhythmia is sensed.

4. A TPMS monitoring apparatus according to any preceding claim in which the processor (22) is adapted to respond to an operator input request to carry out QT analysis or rhythm analysis and to follow fifth or sixth algorithms to perform the requested analysis.

5. A TPMS monitoring apparatus according to claim 4 wherein TPMS monitoring apparatus comprises a display (26) the data store (24) is adapted to contain causal data and values relating to the causes of cardiac arrythmia and related pacing device settings appropriate for each cause, the processor (22) being adapted when requested to perform rhythm analysis to compare instantaneous values with those in the data store, identify the cause which best matches the causal data and values in the data store and to display related pacing device settings.

6. A TPMS monitoring apparatus according to any preceding claim further comprising a visual and or audible alarm to alert an operator/medic/nurse that the functioning of the heart and/or the pacing device is incorrect.

7. A TPMS monitoring apparatus according to any preceding claim further comprising a communications module (28) adapted to communicate to/from a user interface which is remote from the TPMS monitoring apparatus.

8. A TPMS monitoring apparatus according to any preceding claim which is configured to be connected to the patient's heart (4) and the cardiac pacing device (6) by electrical connections such that the heart, the TPMS monitoring apparatus and the pacing device are connected in an inline arrangement.

9. A TPMS monitoring apparatus according to any preceding claim, further comprising electrical connections with the heart (4) and with the pacing device (6), wherein the electrical connections to the heart are placed so as to provide pacing signals to one of or to both of the atrial and ventricular chambers.

10. A TPMS monitoring apparatus according to any preceding claim in which the processor (22) is adapted to integrate and utilise data from additional biomedical sensors.

11. A TPMS monitoring apparatus according to any preceding claim, wherein following the first predetermined algorithm comprises determining that there has been oversensing if:
the processor detects a noise signal; and
the processor either receives not cardiac signal within an oversensing time window following a pacing signal, or receives a plurality of cardiac signals at a rate below a pacing base rate.

12. A TPMS monitoring apparatus according to any preceding claims, wherein following the second predetermined algorithm comprises determining that there has been loss of capture if no evoked response is received within a loss of capture time window following the receipt of a paced impulse.

13. A computer readable medium storing computer instructions that when executed by a processor that is located externally to a patient's body and that is connected to the patient's heart via epicardial or endocardial leads, cause the processor to:
acquire, via electrical connections with the heart and with a pacing device located externally to the patient's body, cardiac signals indicative of cardiac operation, pacing impulses emitted by the pacing device, evoked signals emitted from the heart in response to the pacing impulses, and any noise signals, the electrical connection with the heart comprising the epicardial or endocardial leads;
analyse the cardiac and evoked signals, the pacing impulses and any noise signals, and:
i. establish a base level operation of the heart and pacing device and to store the associated quality, size and/or timing values of the cardiac and evoked signals and the pacing impulses in a data store;
ii. receive instantaneous quality, size and/or timing values of the cardiac and evoked signals and of the pacing impulses;
iii. compare the instantaneous values against the values in the data store to establish differences therebetween;
iv. analyse any difference(s) at step iii above in terms of its/their quality, size and timing and:
follow a first predetermined algorithm in the event a noise signal is received to determine whether or not there has been any oversensing, and/or
follow a second predetermined algorithm in the event that no cardiac or evoked signal is received following a pacing impulse, to determine whether or not there has been loss of capture; and
v. raise an alarm if it is determined that there has been any oversensing and/or any loss of capture.

## Patentansprüche

1. Temporäres Schrittmachervorrichtung, TPMS, - Management- und Sicherheitsüberwachungsgerät (2) zur Überwachung eines temporären Herzschrittmachers (6), der sich außerhalb des Körpers eines Patienten befindet, wobei das TMPS-Überwachungsgerät über epikardiale oder endokardiale Elektroden (10, 14) mit dem Herzen (4) des Patienten verbunden werden kann, wobei sich das TPMS-Überwachungsgerät außerhalb des Körpers des Patienten befindet und umfassend:
ein Signalerfassungsmodul (20), das dazu ausgelegt ist, über elektrische Verbindungen mit dem Herzen und der Schrittmachervorrichtung Herzsignale, die die Herzfunktion anzeigen, von der Schrittmachervorrichtung abgegebene Schrittmacherimpulse, vom Herzen als Reaktion auf die Schrittmacherimpulse abgegebene evozierte Signale und alle nicht identifizierten Rauschsignale zu erfassen, die elektrische Verbindung mit dem Herzen umfassend die epikardialen oder endokardialen Elektroden;
einen Prozessor (22), der dazu ausgelegt ist, vom Signalerfassungsmodul die Herz- und evozierten Signale, die Stimulationsimpulse und etwaige Rauschsignale zu empfangen und zu analysieren; und
einen Datenspeicher (24);
wobei der Prozessor dazu ausgelegt ist:
i. einen Basisbetrieb des Herzens und der Schrittmachervorrichtung festzulegen und die zugehörigen Qualitäts-, Größen- und/oder Zeitwerte der Herz- und evozierten Signale sowie der Schrittmacherimpulse im Datenspeicher zu speichern;
ii. Momentane Qualitäts-, Größen- und/oder Zeitwerte der Herz- und evozierten Signale sowie der Stimulationsimpulse zu empfangen;
iii. die Momentanwerte mit den Werten im Datenspeicher zu vergleichen, um Unterschiede zwischen ihnen festzustellen;
iv. alle Unterschiede in Schritt iii oben hinsichtlich ihrer Qualität, Größe und ihres Zeitpunkts zu analysieren und:
im Falle des Empfangs eines Rauschsignals einem ersten vorgegebenen Algorithmus zu folgen, um zu bestimmen, ob eine Übersensibilisierung vorliegt oder nicht, und/oder
im Falle, dass nach einem Stimulationsimpuls kein Herz- oder evoziertes Signal empfangen wird, einem zweiten vorbestimmten Algorithmus zu folgen, um zu bestimmen, ob ein Verlust der Erfassung vorliegt; und
v. einen Alarm auszulösen, wenn er feststellt, dass eine Übersensibilisierung und/oder ein Verlust der Erfassung vorliegt.

2. TPMS-Überwachungsgerät nach Anspruch 1, bei dem der Prozessor (22) so ausgelegt ist, dass er einem dritten vorbestimmten Algorithmus folgt, wenn ein zeitlich unpassendes Stimulationssignal in Bezug auf ein Herzsignal festgestellt wird, um festzustellen, dass eine Untererfassung vorliegt, bevor ein Alarm ausgelöst wird.

3. TPMS-Überwachungsgerät nach einem der vorstehenden Ansprüche, bei dem der Prozessor (22) so ausgelegt ist, dass er einem vierten Algorithmus folgt, um Arrhythmien zu erkennen, und einen Alarm auslöst, wenn eine Arrhythmie erkannt wird.

4. TPMS-Überwachungsgerät nach einem der vorstehenden Ansprüche, bei dem der Prozessor (22) so ausgelegt ist, dass er auf eine Eingabeanforderung eines Bedieners reagiert, um eine QT-Analyse oder Rhythmusanalyse durchzuführen, und dabei den fünften oder sechsten Algorithmus befolgt, um die angeforderte Analyse durchzuführen.

5. TPMS-Überwachungsgerät nach Anspruch 4, wobei das TPMS-Überwachungsgerät eine Anzeige (26) umfasst, wobei der Datenspeicher (24) dazu ausgelegt ist, Kausaldaten und Werte in Bezug auf die Ursachen von Herzrhythmusstörungen und zugehörige, für jede Ursache geeignete Einstellungen der Schrittmachervorrichtung zu enthalten, wobei der Prozessor (22) dazu ausgelegt ist, bei Anforderung einer Rhythmusanalyse die Momentanwerte mit denen im Datenspeicher zu vergleichen, die Ursache zu identifizieren, die am besten mit den Kausaldaten und Werten im Datenspeicher übereinstimmt, und die zugehörigen Einstellungen des Schrittmachers anzuzeigen.

6. TPMS-Überwachungsgerät nach einem der vorstehenden Ansprüche, umfassend einen optischen und/oder akustischen Alarm, um einen Bediener/Arzt/Krankenpfleger darauf aufmerksam zu machen, dass die Funktion des Herzens und/oder der Schrittmachervorrichtung nicht korrekt ist.

7. TPMS-Überwachungsgerät nach einem der vorstehenden Ansprüche, ferner umfassend ein Kommunikationsmodul (28), das dazu ausgelegt ist, mit einer Benutzeroberfläche zu kommunizieren, die von dem TPMS-Überwachungsgerät entfernt ist.

8. TPMS-Überwachungsgerät nach einem der vorstehenden Ansprüche, das so konfiguriert ist, dass es über elektrische Verbindungen mit dem Herzen (4) des Patienten und der Herzschrittmachervorrichtung (6) verbunden ist, sodass das Herz, das TPMS-Überwachungsgerät und die Schrittmachervorrichtung in einer Inline-Anordnung verbunden sind.

9. TPMS-Überwachungsgerät nach einem der vorhergehenden Ansprüche, das ferner elektrische Verbindungen mit dem Herzen (4) und der Schrittmachervorrichtung (6) umfasst, wobei die elektrischen Verbindungen mit dem Herzen so angeordnet sind, dass sie Schrittmachersignale an eine der Vorhof- und Kammern oder an beide liefern.

10. TPMS-Überwachungsgerät nach einem der vorstehenden Ansprüche, wobei der Prozessor (22) so ausgelegt ist, dass er Daten von zusätzlichen biomedizinischen Sensoren integriert und nutzt.

11. TPMS-Überwachungsgerät nach einem der vorstehenden Ansprüche, wobei der erste vorbestimmte Algorithmus umfasst, dass eine Übersensibilisierung festgestellt wird, wenn:
der Prozessor ein Rauschsignal erkennt; und
der Prozessor entweder innerhalb eines Übersensibilisierung-Zeitfensters nach einem Schrittmachersignal kein Herzsignal empfängt oder eine Vielzahl von Herzsignalen mit einer Frequenz unterhalb einer Schrittmacher-Grundfrequenz empfängt.

12. TPMS-Überwachungsgerät nach einem der vorstehenden Ansprüche, wobei der zweite vorbestimmte Algorithmus umfasst, dass ein Verlust der Erfassung festgestellt wird, wenn innerhalb eines Zeitfensters für den Verlust der Erfassung nach dem Empfang eines Stimulationsimpulses keine evozierte Antwort empfangen wird.

13. Computerlesbares Medium, das Computerbefehle speichert, die, wenn sie von einem Prozessor ausgeführt werden, der sich außerhalb des Körpers eines Patienten befindet und über epikardiale oder endokardiale Leitungen mit dem Herzen des Patienten verbunden ist, den Prozessor dazu veranlassen:
über elektrische Verbindungen mit dem Herzen und mit einer außerhalb des Körpers des Patienten befindlichen Schrittmachervorrichtung Herzsignale, die auf die Herzfunktion hinweisen, von der Schrittmachervorrichtung abgegebene Schrittmacherimpulse, vom Herzen als Reaktion auf die Schrittmacherimpulse abgegebene evozierte Signale und etwaige Rauschsignale zu erfassen, die elektrische Verbindung mit dem Herzen umfassend die epikardialen oder endokardialen Elektroden;
die Herz- und evozierten Signale, die Stimulationsimpulse und etwaige Rauschsignale zu analysieren und:
i. einen Basisbetrieb der Herz- und Schrittmachervorrichtung festzulegen und die zugehörigen Qualitäts-, Größen- und/oder Zeitwerte der Herz- und evozierten Signale sowie der Schrittmacherimpulse in einem Datenspeicher zu speichern;
ii. Momentane Qualitäts-, Größen- und/oder Zeitwerte der Herz- und evozierten Signale sowie der Stimulationsimpulse zu empfangen;
iii. die Momentanwerte mit den Werten im Datenspeicher zu vergleichen, um Unterschiede zwischen ihnen festzustellen;
iv. alle Unterschiede in Schritt iii oben hinsichtlich ihrer Qualität, Größe und ihres Zeitpunkts zu analysieren und:
einen ersten vorgegebenen Algorithmus zu befolgen, falls ein Rauschsignal empfangen wird, um festzustellen, ob eine Übersensibilisierung vorliegt oder nicht, und/oder
einen zweiten vorbestimmten Algorithmus zu befolgen, falls nach einem Stimulationsimpuls kein Herz- oder evoziertes Signal empfangen wird, um festzustellen, ob ein Erfassungsverlust vorliegt oder nicht; und
v. einen Alarm auszulösen, wenn festgestellt wird, dass eine Übersensibilisierung und/oder ein Verlust der Erfassung vorliegt.

## Revendications

1. Appareil de surveillance de gestion et de sécurité de stimulation temporaire, TPMS, (2) adapté à la surveillance d'un dispositif de stimulation cardiaque temporaire (6) qui est situé à l'extérieur du corps d'un patient, l'appareil de surveillance TMPS pouvant être connecté au cœur du patient (4) via des sondes épicardiques ou endocardiques (10, 14), l'appareil de surveillance TPMS étant situé à l'extérieur du corps du patient et comprenant :
un module d'acquisition de signal (20) adapté pour acquérir via des connexions électriques avec le cœur et le dispositif de stimulation, des signaux cardiaques indicatifs du fonctionnement cardiaque, des impulsions de stimulation émises par le dispositif de stimulation, des signaux évoqués émis par le cœur en réponse aux impulsions de stimulation, et tous signaux de bruit non identifiés, la connexion électrique avec le cœur comprenant les sondes épicardiques ou endocardiques ;
un processeur (22) adapté pour recevoir en provenance du module d'acquisition de signal et pour analyser les signaux cardiaques et évoqués, les impulsions de stimulation et tous signaux de bruit ; et
une mémoire de données (24) ;
dans lequel le processeur est adapté pour :
i. établir un fonctionnement de niveau de base du cœur et du dispositif de stimulation et pour stocker les valeurs associées de qualité, de dimension et/ou de synchronisation des signaux cardiaques et évoqués et des impulsions de stimulation dans la mémoire de données ;
ii. recevoir des valeurs instantanées de qualité, de dimension et/ou de synchronisation de signaux cardiaques et évoqués et des impulsions de stimulation ;
iii. comparer les valeurs instantanées aux valeurs dans la mémoire de données pour établir les différences entre elles ;
iv. analyser toute différence à l'étape iii ci-dessus en termes de qualité, de dimension et de synchronisation et :
dans le cas où un signal de bruit est reçu, suivre un premier algorithme prédéterminé pour déterminer s'il y a eu une surdétection ou non, et/ou
dans le cas où aucun signal cardiaque ou évoqué n'est reçu à la suite d'une impulsion de stimulation, suivre un deuxième algorithme prédéterminé pour déterminer s'il y a eu une perte de capture ou non ; et
v. déclencher une alarme s'il détermine qu'il y a eu une surdétection et/ou une perte de capture.

2. Appareil de surveillance TPMS selon la revendication 1 dans lequel le processeur (22) est adapté pour suivre un troisième algorithme prédéterminé lorsqu'un signal de stimulation synchronisé de manière inappropriée est observé par rapport à un signal cardiaque pour établir qu'il y a eu une sous-détection avant qu'une alarme ne soit déclenchée.

3. Appareil de surveillance TPMS selon une quelconque revendication précédente dans lequel le processeur (22) est adapté pour suivre un quatrième algorithme pour détecter l'arythmie, et déclencher une alarme lorsque l'arythmie est détectée.

4. Appareil de surveillance TPMS selon une quelconque revendication précédente dans lequel le processeur (22) est adapté pour répondre à une demande d'entrée d'opérateur pour exécuter une analyse QT ou une analyse de rythme et pour suivre un cinquième ou un sixième algorithme pour réaliser l'analyse demandée.

5. Appareil de surveillance TPMS selon la revendication 4 dans lequel l'appareil de surveillance TPMS comprend un affichage (26) la mémoire de données (24) est adaptée pour contenir des données causales et des valeurs relatives aux causes de l'arythmie cardiaque et relatives aux paramètres de dispositif de stimulation appropriés à chaque cause, le processeur (22) étant adapté sur demande pour réaliser une analyse de rythme pour comparer les valeurs instantanées avec celles dans la mémoire de données, identifier la cause qui correspond le mieux aux données causales et aux valeurs dans la mémoire de données et pour afficher les paramètres de dispositif de stimulation associés.

6. Appareil de surveillance TPMS selon une quelconque revendication précédente comprenant en outre une alarme visuelle et/ou sonore pour alerter un opérateur/médecin/personnel soignant que le fonctionnement du cœur et/ou du dispositif de stimulation est incorrect.

7. Appareil de surveillance TPMS selon une quelconque revendication précédente comprenant en outre un module de communication (28) adapté pour communiquer avec une interface utilisateur qui est distante de l'appareil de surveillance TPMS.

8. Appareil de surveillance TPMS selon une quelconque revendication précédente qui est configuré pour être connecté au cœur du patient (4) et au dispositif de stimulation cardiaque (6) par des connexions électriques de telle sorte que le cœur, l'appareil de surveillance TPMS et le dispositif de stimulation sont connectés dans un agencement en ligne.

9. Appareil de surveillance TPMS selon une quelconque revendication précédente, comprenant en outre des connexions électriques avec le cœur (4) et avec le dispositif de stimulation (6), dans lequel les connexions électriques au cœur sont placées de manière à fournir des signaux de stimulation à l'une ou aux deux des chambres auriculaires et ventriculaires.

10. Appareil de surveillance TPMS selon une quelconque revendication précédente dans lequel le processeur (22) est adapté pour intégrer et utiliser des données provenant de capteurs biomédicaux supplémentaires.

11. Appareil de surveillance TPMS selon une quelconque revendication précédente, dans lequel le fait de suivre le premier algorithme prédéterminé comprend la détermination qu'il y a eu une surdétection si :
le processeur détecte un signal de bruit ; et
le processeur ne reçoit aucun signal cardiaque dans une fenêtre temporelle de surdétection suivant un signal de stimulation, ou reçoit une pluralité de signaux cardiaques à une fréquence inférieure à une fréquence de base de stimulation.

12. Appareil de surveillance TPMS selon l'une quelconque des revendications précédentes, dans lequel le fait de suivre le deuxième algorithme prédéterminé comprend la détermination qu'il y a eu perte de capture si aucune réponse évoquée n'est reçue dans une fenêtre temporelle de perte de capture suivant la réception d'une impulsion stimulée.

13. Support lisible par ordinateur stockant des instructions informatiques qui lorsqu'elles sont exécutées par un processeur qui est situé à l'extérieur du corps du patient et qui est relié au cœur du patient via des sondes épicardiques ou endocardiques, amènent le processeur à :
acquérir, via des connexions électriques avec le cœur et avec un dispositif de stimulation situé à l'extérieur du corps du patient, des signaux cardiaques indicatifs du fonctionnement cardiaque, des impulsions de stimulation émises par le dispositif de stimulation, des signaux évoqués émis par le cœur en réponse aux impulsions de stimulation, et tous signaux de bruit, la connexion électrique avec le cœur comprenant les sondes épicardiques ou endocardiques ;
analyser les signaux cardiaques et évoqués, les impulsions de stimulation et tous signaux de bruit, et :
i. établir un fonctionnement de niveau de base du cœur et du dispositif de stimulation et stocker les valeurs associées de qualité, de dimension et/ou de synchronisation des signaux cardiaques et évoqués et des impulsions de stimulation dans une mémoire de données ;
ii. recevoir des valeurs instantanées de qualité, de dimension et/ou de synchronisation de signaux cardiaques et évoqués et des impulsions de stimulation ;
iii. comparer les valeurs instantanées aux valeurs dans la mémoire de données pour établir les différences entre elles ;
iv. analyser toute différence à l'étape iii ci-dessus en termes de qualité, de dimension et de synchronisation et :
suivre un premier algorithme prédéterminé dans le cas où un signal de bruit est reçu pour déterminer s'il y a eu une surdétection ou non, et/ou
suivre un deuxième algorithme prédéterminé dans le cas où aucun signal cardiaque ou évoqué n'est reçu à la suite d'une impulsion de stimulation, pour déterminer s'il y a eu une perte de capture ou non ; et
v. déclencher une alarme s'il est déterminé qu'il y a eu une surdétection et/ou une perte de capture.
